(19) Europäisches Patentamt
European Patent Office
Office européen des brevets

(11) **EP 2 158 880 B1**

(12) **EUROPEAN PATENT SPECIFICATION**

(45) Date of publication and mention
of the grant of the patent:
**18.04.2012  Bulletin 2012/16**

(51) Int Cl.:
***A61F 2/90*** *(2006.01)*

(21) Application number: **09168241.9**

(22) Date of filing: **20.08.2009**

(54) **Intravascular stent having improved design for loading and deploying**

Intravaskulärer Stent mit verbesserter Gestaltung zum Beladen und Anwenden

Endoprothèse intravasculaire dotée d'un design amélioré pour le chargement et le déploiement

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **26.08.2008  US 198150**

(43) Date of publication of application:
**03.03.2010  Bulletin 2010/09**

(73) Proprietor: **Cordis Corporation**
**Miami Lakes, FL 33014 (US)**

(72) Inventors:
• **Caldarise, Salvatore G.**
**Belle Mead, NJ 08502 (US)**

• **Majercak, David C.**
**Stewartsville, NJ 08886 (US)**

(74) Representative: **Brown, George Laurence**
**Urquhart-Dykes & Lord LLP**
**Tower North Central**
**Merrion Way**
**Leeds LS2 8PA (GB)**

(56) References cited:
**EP-A1- 1 886 650      EP-A2- 1 184 007**
**WO-A2-02/24111      WO-A2-2008/070304**

**Description**

The present invention relates to stents for use within a body passageway or duct and more particularly to stents having improved strut designs for improved durability performance without sacrificing loading and deployment ease.

Percutaneous transluminal coronary angioplasty (PTCA) is a therapeutic medical procedure used to increase blood flow through the coronary arteries and may often be used as an alternative to coronary bypass surgery. In this procedure, an angioplasty balloon is inflated within the stenosed vessel, or body passageway, in order to shear and disrupt the wall components of the vessel to obtain an enlarged lumen. With respect to arterial stenosed lesions, the relatively incompressible plaque remains unaltered, while the more elastic medial and adventitial layers of the body passageway stretch around the plaque. This process typically produces dissection, or a splitting and tearing, of the body passageway wall layers, wherein the intima, or internal surface of the artery or body passageway, suffers fissuring. This dissection forms a "flap", of underlying tissue which may reduce the blood flow through the lumen, or block the lumen. Typically, the distending intraluminal pressure within the body passageway can hold the disrupted layer, or flap, in place. If the intimal flap created by the balloon dilation procedure is not maintained in place against the expanded intima, the intimal flap can fold down into the lumen and close off the lumen, or may even become detached and enter the body passageway. When the intimal flap closes off the body passageway, immediate surgery is necessary to correct the problem.

Recently, transluminal prostheses have been widely used in the medical arts for implantation in blood vessels, biliary ducts, or other similar organs of the living body. These prostheses are commonly known as stents and are used to maintain, open, or dilate tubular structures. An example of a commonly used stent is given in US-4,733,665 filed by Palmaz on Nov. 7, 1985. Such stents are often referred to as balloon expandable stents. Typically the stent is made from a solid tube of stainless steel. Thereafter, a series of cuts are made in the wall of the stent. The stent has a first smaller diameter which permits the stent to be delivered through the human vasculature by being crimped onto a balloon catheter. The stent also has a second, expanded diameter, upon the application, by a balloon catheter, from the interior of the tubular shaped member of a radially, outwardly extending force.

EP 1 184 007 discloses a stent with struts having a variable width.

However, such stents are often impractical for use in some vessels such as the carotid artery. The carotid artery is easily accessible from the exterior of the human body, and is often visible by looking at ones neck. A patient having a balloon expandable stent made from stainless steel or the like, placed in their carotid artery might be susceptible to sever injury through day to day activity. A sufficient force placed on the patients neck, such as by falling, could cause the stent to collapse, resulting in injury to the patient. In order to prevent this, self expanding stents have been proposed for use in such vessels. Self expanding stents act like springs and will recover to their expanded or implanted configuration after being crushed.

One type of self-expanding stent is disclosed in US-4,665,771, which stent has a radially and axially flexible, elastic tubular body with a predetermined diameter that is variable under axial movement of ends of the body relative to each other and which comprises of a plurality of individually rigid but flexible and elastic thread elements defining a radially self-expanding helix. This type of stent is known in the art as a "braided stent" and is so designated herein. Placement of such stents in a body vessel may be achieved by a device which comprise an outer catheter for holding the stent at its distal end, and an inner piston which pushes the stent forward once it is in position.

However, braided stents have a number of disadvantages. They typically do not have the necessary radial strength to effectively hold open a diseased vessel. In addition, the plurality of wires or fibers used to make such stents could become dangerous if separated from the body of the stent, where it could pierce through the vessel. Therefore, there has been a desire to have a self-expanding stent, which is cut from a tube of metal, which is the common manufacturing method for many commercially available balloon expandable stents. In order to manufacture a self-expanding stent cut from a tube, the alloy used would preferably have superelastic or psuedoelastic characteristics at body temperature, so that it is crush recoverable.

The prior art makes reference to the use of alloys such as Nitinol (Ni-Ti alloy) which have shape memory and/or superelastic characteristics in medical devices which are designed to be inserted into a patient's body. The shape memory characteristics allow the devices to be deformed to facilitate their insertion into a body lumen or cavity and then be heated within the body so that the device returns to its original shape. Superelastic characteristics on the other hand generally allow the metal to be deformed and restrained in the deformed condition to facilitate the insertion of the medical device containing the metal into a patient's body, with such deformation causing the phase transformation. Once within the body lumen the restraint on the superelastic member may be removed, thereby reducing the stress therein so that the superelastic member can return to its original un-deformed shape by the transformation back to the original phase.

Alloys having shape memory/superelastic characteristics generally have at least two phases. These phases are a martensite phase, which has a relatively low tensile strength and which is stable at relatively low temperatures, and an austenite phase, which has a relatively high tensile strength and which is stable at temperatures higher than the martensite phase.

Shape memory characteristics are imparted to the alloy by heating the metal at a temperature above which the transformation from the martensite phase to the austenite phase is complete, i.e. a temperature above which the austenite phase is stable (the Af temperature). The shape of the metal during this heat treatment is the shape "remembered." The heat treated metal is cooled to a temperature at which the martensite phase is stable, causing the austenite phase to transform to the martensite phase. The metal in the martensite phase is then plastically deformed, e.g. to facilitate the entry thereof into a patient's body. Subsequent heating of the deformed martensite phase to a temperature above the martensite to austenite transformation temperature causes the deformed martensite phase to transform to the austenite phase and during this phase transformation the metal reverts back to its original shape if unrestrained. If restrained, the metal will remain martensitic until the restraint is removed.

Methods of using the shape memory characteristics of these alloys in medical devices intended to be placed within a patient's body present operational difficulties. For example, with shape memory alloys having a stable martensite temperature below body temperature, it is frequently difficult to maintain the temperature of the medical device containing such an alloy sufficiently below body temperature to prevent the transformation of the martensite phase to the austenite phase when the device was being inserted into a patient's body. With intravascular devices formed of shape memory alloys having martensite-to-austenite transformation temperatures well above body temperature, the devices may be introduced into a patient's body with little or no problem, but they must be heated to the martensite-to-austenite transformation temperature which is frequently high enough to potentially cause tissue damage and very high levels of pain.

When stress is applied to a specimen of a metal such as Nitinol exhibiting superelastic characteristics at a temperature above which the austenite is stable (i.e. the temperature at which the transformation of martensite phase to the austenite phase is complete), the specimen deforms elastically until it reaches a particular stress level where the alloy then undergoes a stress-induced phase transformation from the austenite phase to the martensite phase. As the phase transformation proceeds, the alloy undergoes significant increases in strain but with little or no corresponding increases in stress. The strain increases while the stress remains essentially constant until the transformation of the austenite phase to the martensite phase is complete. Thereafter, further increases in stress are necessary to cause further deformation. The martensitic metal first deforms elastically upon the application of additional stress and then plastically with permanent residual deformation.

If the load on the specimen is removed before any permanent deformation has occurred, the martensitic specimen will elastically recover and transform back to the austenite phase. The reduction in stress first causes a decrease in strain. As stress reduction reaches the level at which the martensite phase transforms back into the austenite phase, the stress level in the specimen will remain essentially constant (but substantially less than the constant stress level at which the austenite transforms to the martensite) until the transformation back to the austenite phase is complete, i.e. there is significant recovery in strain with only negligible corresponding stress reduction. After the transformation back to austenite is complete, further stress reduction results in elastic strain reduction. This ability to incur significant strain at relatively constant stress upon the application of a load and to recover from the deformation upon the removal of the load is commonly referred to as superelasticity or pseudoelasticity. It is this property of the material which makes it useful in manufacturing tube cut, self-expanding stents. The prior art makes reference to the use of metal alloys having superelastic characteristics in medical devices which are intended to be inserted or otherwise used within a patient's body. See for example, US- 4,665,905 (Jervis) and US- 4,925,445 (Sakamoto et al.).

However, the prior art has yet to disclose ideal tube cut, self expanding stents. In addition, a number of the prior art stents lacked the necessary rigidity or hoop strength to keep the body vessel open. In addition, a number of the prior art stents have large openings at their expanded diameter. The smaller the openings are on an expanded stent, the more plaque or other deposits it can trap between the stent and the vessel wall. Trapping these deposits is important to the continuing health of the patient in that it helps prevent stokes as well as helps prevents restenosis of the vessel it is implanted into. In addition, many of the prior art stents have failed to optimize stent mechanical performance characteristics relative to their size for percutaneous delivery.

The present invention overcomes the difficulties as briefly described above.

The present invention is directed to an intraluminal device for maintaining vessel patency. The device comprising a substantially tubular structure having a first diameter for insertion into a vessel and a second diameter for deployment in a vessel, the substantially tubular structure being formed from a plurality of hoops, wherein adjacent hoops are connected by one or more bridges, each hoop comprising a plurality of longitudinally arranged struts, each strut having two opposing ends and a center therebeween, the width of the struts being greater at its opposing ends than at its center, and a plurality of loops connecting the plurality of struts to form a substantially s-shaped pattern, and wherein one or more struts in each hoop comprises an one or more circumferentially extending protrusions proximate the center thereof.

The present invention utilizes small additional elements or features added to tapered struts of a stent to increase the pushability of the stent during the process of loading the stent into a delivery device, and deployment of the stent without complicating delivery system features. For example, the delivery of a highly flexible stent may require the utilization of stent retention and securement features built into one or both the stent and the stent delivery system, such as maturing and/or interlocking features, which by necessity, would have to be aligned with one another. As described in detail herein, tapered struts offer an advantage over straight struts by distributing the stress over the length of the strut, and thus minimize local stress concentrations near the apex or loop of the strut. This improved distribution results in an increase in stent fatigue resistance. Also described in detail herein, while stent flexibility is paramount and may be optimized/achieved with tapered struts, use of tapered struts may potentially result in buckling during stent loading into the delivery system. Accordingly, the present invention utilizes added features to the tapered struts that stabilize the gaps between struts when the stent is loaded into the delivery system. These additional features solve the problem without adversely affecting the stress distribution. In fact, slight improvement in fatigue resistance occurs when these features are incorporated as part of the tapered strut as confirmed using finite element analysis.

The present invention is a simple solution to the potential buckling problem associated with loading. The additional elements are easily cut as part of the overall manufacturing process and have no known adverse effects on stent performance.

Embodiments of the invention will now be described by way of example with reference to the accompanying drawings, in which:

Figure 1 is a simplified partial cross-sectional view of a stent delivery apparatus having a stent loaded therein, which can be used with a stent made in accordance with the present invention.

Figure 2 is a view similar to that of Figure 1 but showing an enlarged view of the distal end of the delivery apparatus in accordance with the present invention.

Figure 3 is a perspective view of a stent made in accordance with the present invention, showing the stent in its compressed state.

Figure 4 is a sectional, flat view of the stent shown in Figure 1.

Figure 4A is an enlarged view of section of the stent shown in Figure 4.

Figure 5 is a perspective view of the stent shown in Figure 1 but showing it in its expanded state.

Figures 6A and 6B are sectional, flat views of a first exemplary embodiment of a stent having modified elements in accordance with the present invention showing the expanded and compressed states/configuration respectfully.

Figure 7 is a sectional, flat view of a second exemplary embodiment of a stent in the expanded state having modified elements in accordance with the present invention.

Figures 8A and 8B are sectional, flat views of a third exemplary embodiment of a stent having modified elements in accordance with the present invention showing the expanded and compressed states/configuration respectfully.

Figure 9 is a sectional, flat view of a fourth exemplary embodiment of a stent in the expanded state having modified elements in accordance with the present invention.

Figures 10A and 10B are sectional, flat views of a fifth exemplary embodiment of a stent having modified elements in accordance with the present invention showing the expanded and compressed states/configuration respectfully.

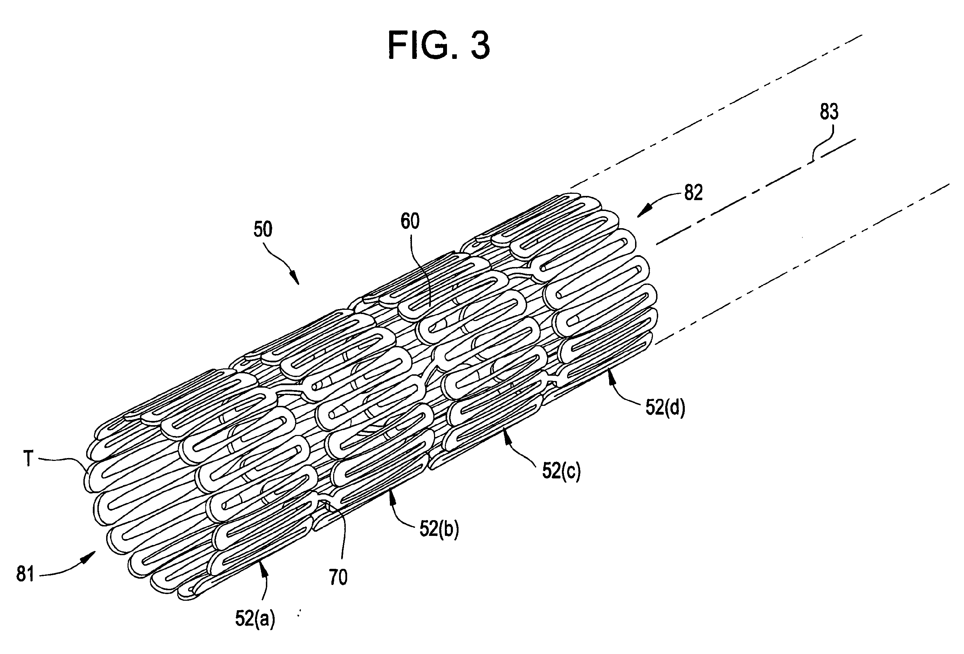

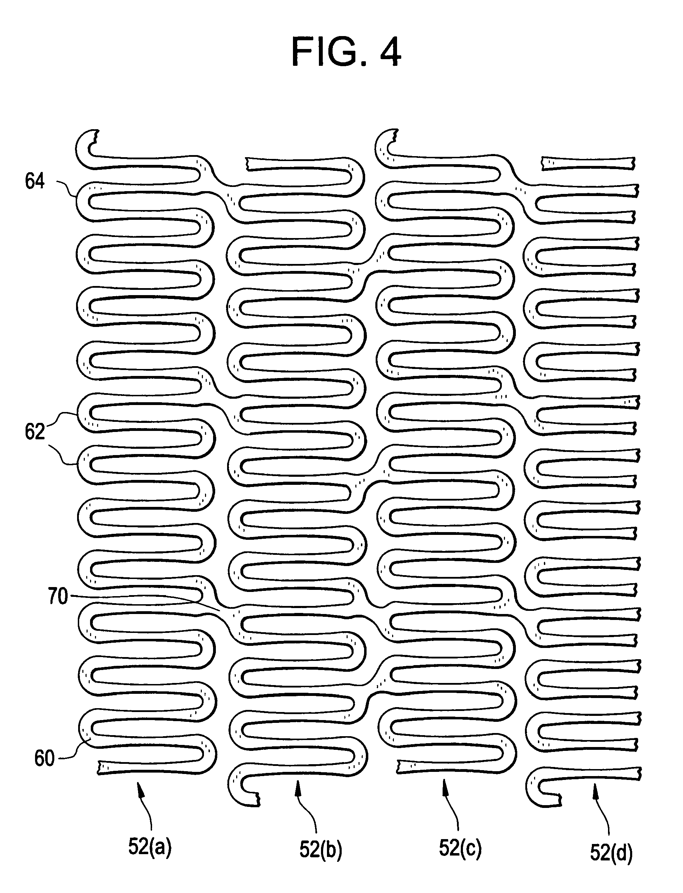

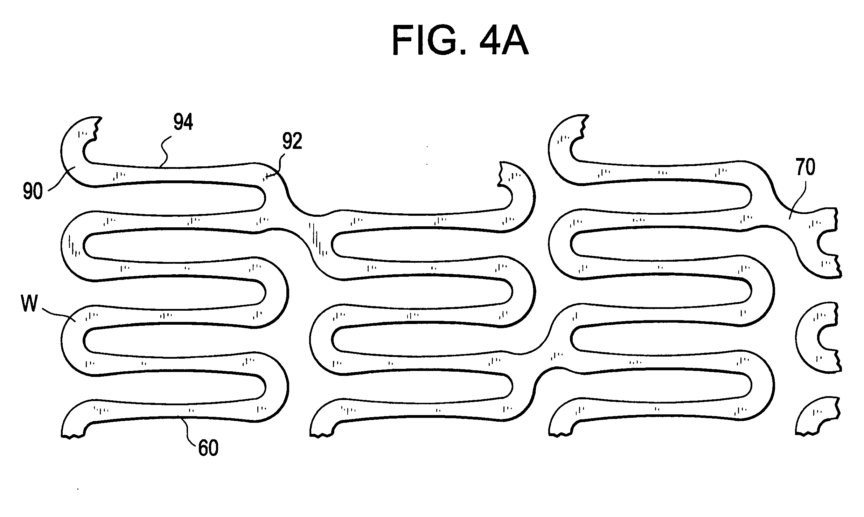

Referring now to the figures wherein like numerals indicate the same element throughout the views, there is shown in Figures 3, 4, and 4A a stent 50 made in accordance with the present invention. Figures 3 and 4 show stent 50 in its un-expanded or compressed state. Stent 50 is preferably made from a superelastic alloy such as Nitinol. Most preferably, stent 50 is made from an alloy comprising from about 50.5 percent (as used herein these percentages refer to atomic percentages) Ni to about 60 percent Ni, and most preferably about 55 percent Ni, with the remainder of the alloy Ti. Preferably, the stent is such that it is superelastic at body temperature, and preferably has an Af in the range from about twenty-four degrees Celsius to about thirty-seven degrees Celsius. The superelastic design of the stent makes it crush recoverable which, as discussed above, can be used as a stent or frame for any number of vascular devices for different applications.

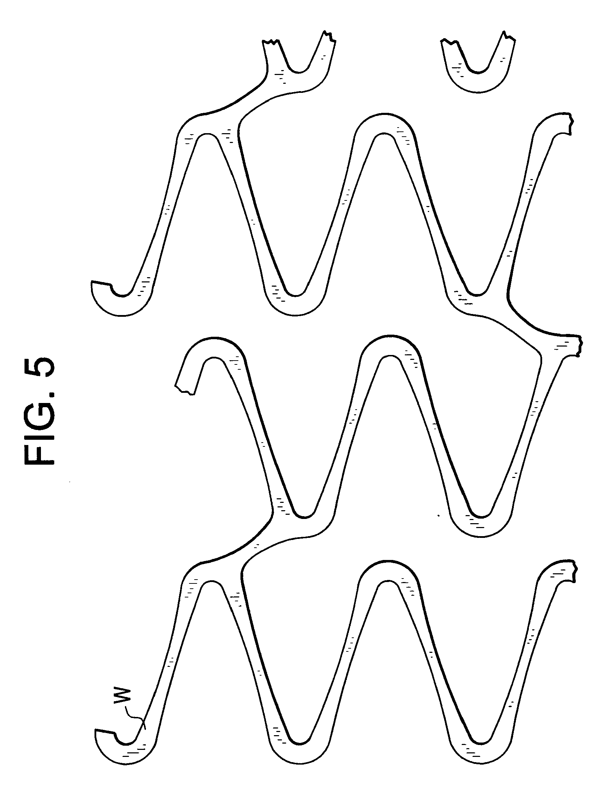

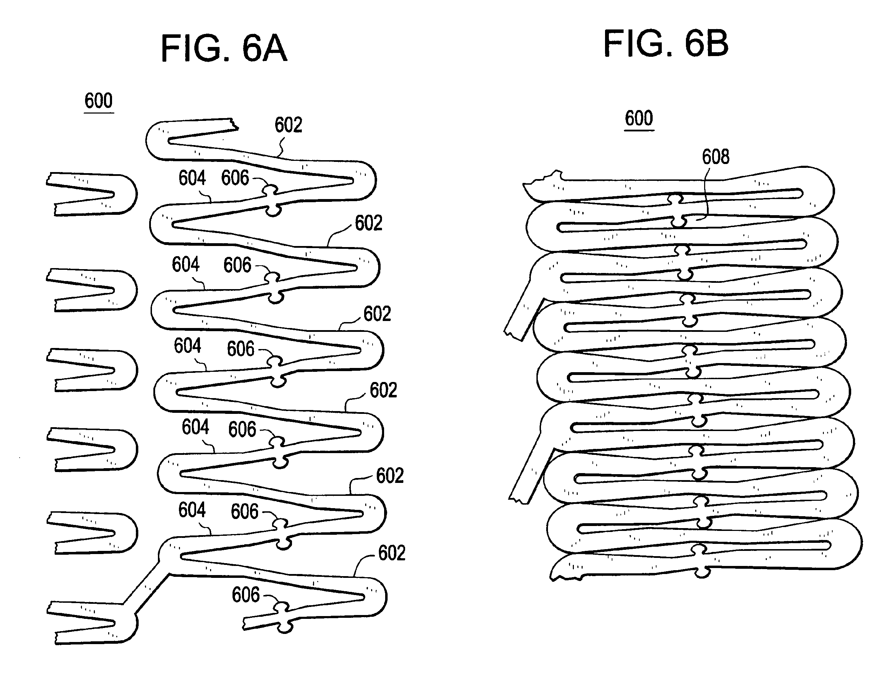

Stent 50 is a tubular member having front and back open ends 81 and 82 and a longitudinal axis 83 extending therebetween. The tubular member has a first smaller diameter, Figures 3 and 4, for insertion into a patient and navigation through the vessels, and a second larger diameter, Figures 5 and 6, for deployment into the target area of a vessel. The tubular member is made from a plurality of adjacent hoops 52, Figure 3 showing hoops 52(a)-52(d), extending between the front and back ends 81 and 82. The hoops 52 include a plurality of longitudinal struts 60. As seen from Figure 4A, each strut 60 has two opposing ends 90 and 92 and a center 94 therebetween. The ends 90 and 92 of the struts 60 are curved or bent so as to form a plurality of loops 62, which connect adjacent struts. The struts are so connected at their opposite ends so as to form an S or Z shape pattern. The loops 62 are preferably curved, substantially semi-circular and symmetrical sections.

Stent 50 further includes a plurality of bridges 70 which connect adjacent hoops 52 and that may best be described by referring to Figure 4. Each bridge 70 has two ends, wherein one end is attached to one strut and/or loop, and another end attached to a strut and/or loop on an adjacent hoop 52. While the Figures show the bridges 70 connecting the loop 62 of one bridge to the nearest loop 62 on the adjacent hoop 52, this does not need to be so. The bridge 70 could be longer and extend the length of many struts between its connection point on adjacent hoops 52. The bridges 70 are curved, and are attached to loops 62 at points off center of the radius of curvature of the loops 62.

The above described geometry helps to better distribute strain throughout the stent, prevents metal to metal contact when the stent is bent, and minimizes the opening size between the features, struts, loops and bridges. The number of and nature of the design of the struts, loops and bridges are important factors when determining the working properties and fatigue life properties of the stent. It was previously thought that in order to improve the rigidity of the stent, that struts should be large, and therefore there should be fewer struts per hoop. However, it has now been discovered that stents having smaller struts and more struts per hoop actually improve the construction of the stent and provide greater rigidity. Preferably, each hoop has between twenty-four to thirty-six or more struts. It has been determined that a stent having a ratio of number of struts 60 per hoop 52 to strut length L (in inches) which is greater than four hundred has increased rigidity over prior art stents which typically had a ratio of under two hundred. The length of a strut 60 is measured in its compressed state parallel to the longitudinal axis 83 of the stent.

The present invention may best be understood by referring back to Figures 4 and 4A. As seen from Figure 4A, each strut has a width W, measured in a substantially circumferential direction, which is greater at its ends 90 and 92, and points adjacent thereto, than in its center 94. Preferably, the width W tapers substantially continuously from each of the ends 90 and 92 to the center 94. The effect of this tapering will be to cause a greater resistance to deformation at the loops 62 (where the bending moments are high), and to make the overall strain deformation more uniform. The ideal reduction in width is a complex function, driven by efforts to keep the bending radius constant. Bending of rectangular beam is controlled by the formula:

$$1/R = 12FL/(ETW^3)$$

where R is the radius of curvature of the loops (to remain constant), F is the applied force, L the distance from the endpoint, E is Young's modulus, T the thickness of the strut (shown in Figure 3) and W the strut width (shown in Figure 4A). Thus as a guideline, the strut width W should vary as the cube root of the distance from either of the ends, 90 or 92. That is, at any point along the center 94 of a strut 60 the width should be proportional to the cube root of the distance from the end point that is closest to strut ends, 90 or 92. However, any taper, even a simple linear tapered reduction in width would still represent a substantial improvement over a constant width strut.

Because the struts are wider at their ends, the overall stent can handle greater compressive and expanding forces. Therefore, stents having smaller delivery diameters and greater expanded diameters can be made while still being extremely flexible over the entire stent length. In addition, the stent can handle greater fatigue stresses, which could result in a longer lasting and stronger stent.

As seen from Figure 5, the geometry of the stent changes quite significantly as a stent is deployed from its un-expanded state to its expanded state. As a stent undergoes diametric change, the strut angle and strain levels in the loops and bridges are effected. Preferably, all of the stent features will strain in a predictable manor so that the stent is reliable and uniform in strength. In addition, it is preferable to minimize the maximum strain experienced by struts loops and bridges, since Nitinol properties are more generally limited by strain, rather than by stress as most materials are. As will be discussed in greater detail below, the stent sits in the delivery system in its un-expanded or compressed state as shown in Figure 3. As the stent is deployed, it is allowed to expand towards it's expanded state, as shown in Figure 5, which preferably has a diameter which is the same or larger than the diameter of the target vessel. Nitinol stents made from wire (as opposed to being cut from a tube) deploy in much the same manor and are dependent upon the same design constraints as laser cut stents. Stainless steel stents deploy similarly in terms of geometric changes although they are assisted with forces from balloons or other devices.

In trying to minimize the maximum strain experienced by the features (struts 60, loops 62 and bridges 70), the present invention utilizes structural geometry's which distribute strain to areas of the stent which are less susceptible to failure than others. For example, one of the most vulnerable areas of the stent is the inside radius of the connecting loops 62. The connecting loops 62 undergo the most deformation of all the stent features. The inside radius of the loop 62 would normally be the area with the highest level of strain on the stent. This area is also critical in that it is usually the smallest radius on the stent. Stress concentrations are generally controlled or minimized by maintaining the largest radii possible, and by dual tapering the width of struts as disclosed above. Similarly, we want to minimize local strain concentrations on the bridge 70 and bridge connection points. One way to accomplish this is to utilize the largest possible radii while maintaining feature widths which are consistent with applied forces. Another consideration is to minimize the maximum open area of the stent. Efficient utilization of the original tube from which the stent is cut increases stent strength and its ability to trap embolic material.

As mentioned above, bridge geometry changes both as a stent is deployed from its compressed state to its expanded state and from its expanded state to a compressed state. As a stent undergoes diametric change, strut angle and loop strain is effected. Since the bridges 70 are connected to either the loops 62, struts 60 or both, they are effected. Twisting of one end of the stent with respect to the other, while loaded in the stent delivery system, should be avoided. Local torque delivered to the bridge ends displaces the bridge geometry. If the bridge design is duplicated around the stent perimeter, this displacement causes rotational shifting of the two hoops being connected by the bridges. If the bridge design is duplicated throughout the stent, this shift will occur down the length of the stent. This is a cumulative effect as one considers rotation of one end with respect to the other upon deployment. A stent delivery system, such as the one described below, will deploy the distal end first, then allow the proximal end to expand. It would be undesirable to allow the distal end to anchor into the vessel wall while holding the stent fixed in rotation, then release the proximal end. In doing so, this could cause the stent to twist or whip in rotation to equilibrium after it is at least partially deployed within the vessel. Such whipping action could cause damage to the vessel.

However, in accordance with an exemplary embodiment of the present invention, as shown in the figures, this design reduces the chance of such events from happening when deploying the stent. By mirroring the bridge geometry longitudinally down the stent, the rotational shift of the Z-sections can be made to alternate and will minimize large rotational changes between any two points on respective hoops 52 on a given stent during deployment or constraint. That is the bridges 70 connecting loop 52(b) to loop 52(c) are angled upwardly from left to right, while the bridges 70 connecting loop 52(c) to loop 52(d) are angled downwardly from left to right. This alternating pattern is repeated down the length of the stent. This alternating pattern of bridge slopes improves the torsional characteristics of the stent so as to minimize any twisting or rotation of the stent with respect to any two hoops 52. This alternating bridge slope is particularly beneficial if the stent starts to twist in vivo. As the stent twists, the diameter of the stent will change. Alternating bridge slopes tend to minimize this effect. The diameter of a stent having bridges 70 which are all sloped in the same direction will tend grow if twisted in one direction and shrink if twisted in the other direction. With alternating bridge slopes this effect is minimized and localized.

The above-described feature is particularly advantageous for stents having large expansion ratios, which in turn requires them to have extreme bending requirements where large elastic strains are required. Nitinol can withstand extremely large amounts of elastic strain deformation, so the above features are well suited to stents made from this alloy. This feature allows for maximum utilization of Ni--Ti or other material capabilities to enhance radial strength, improve stent strength uniformity, improve fatigue life by minimizing local strain levels, allow for smaller open areas which enhance entrapment of embolic material, and improve stent apposition in irregular vessel wall shapes and curves.

Preferably, stents are laser cut from small diameter tubing. For prior art stents, this manufacturing process lead to designs with geometric features, such as struts, loops and bridges, having axial widths which are larger than the tube wall thickness T (shown in Figure 3). When the stent is compressed, most of the bending occurs in the plane that is created if one were to cut longitudinally down the stent and flatten it out. However, for the individual bridges, loops and struts, which have widths greater than their thickness, they have a greater resistance to this in-plane bending than they do to out of plane bending. Because of this, the bridges and struts tend to twist, so that the stent as a whole can bend more easily. This twisting is a buckling condition which is unpredictable and can cause potentially high strain.

However, this problem has been solved in a preferred exemplary embodiment of the present invention, shown in the figures. For the present invention, it is preferred that the maximum widths of the struts 60, hoops 52 and bridges 70 are equal to or less than the wall thickness of the tube. Therefore, substantially all bending and, therefore, all strains are "out of plane." This minimizes twisting of the stent which minimizes or eliminates buckling and unpredictable strain conditions. The feature is particularly advantageous for stents having large expansion ratios, which in turn requires them to have extreme bending requirements where large elastic strains are required. Nitinol can withstand extremely large amounts of elastic strain deformation, so the above features are well suited to stents made from this alloy as described above.

While the current invention may be either a self expanding or balloon expandable stent, and may be made from any number of materials known in the art, including stainless steel, as mentioned above, it is preferred that the stent of the present invention be made from a superelastic alloy and most preferably made of an alloy material having greater than 50.5 atomic percent Nickel and the balance titanium. Greater than 50.5 atomic percent Nickel allows for an alloy in which the temperature at which the martensite phase transforms completely to the austenite phase (the Af temperature) is below human body temperature and preferably is about twenty-four degrees Celsius to about thirty-seven degrees Celsius so that austenite is the only stable phase at body temperature.

In manufacturing the Nitinol stent, the material is first in the form of a tube. Nitinol tubing is commercially available from a number of suppliers. The tubular member is then loaded into a machine which will cut the predetermined pattern of the stent, which was discussed above and is shown in the Figures, into the tube. Machines for cutting patterns in tubular devices to make stents or the like are well known to those of ordinary skill in the art and are commercially available. Such machines typically hold the metal tube between the open ends while a cutting laser, preferably under microprocessor control, cuts the pattern. The pattern dimensions and styles, laser positioning requirements, and other information are programmed into a microprocessor which controls all aspects of the process. After the stent pattern is cut, the stent is treated and polished using any number of methods well known to those skilled in the art. Lastly, the stent is then cooled until it is completely martensitic, crimped down to its un-expanded diameter and then loaded into the sheath of the delivery apparatus.

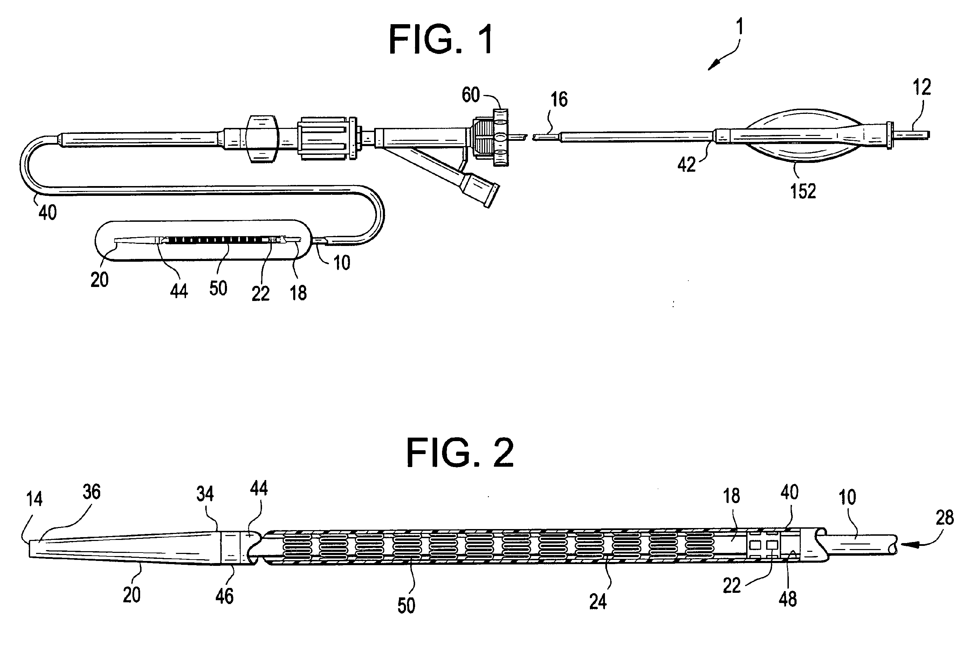

It is believed that many of the advantages of the present invention may be better understood through a brief description of a delivery apparatus for the stent, as shown in Figures 1 and 2. Figures 1 and 2 show a self-expanding stent delivery apparatus 1 for a stent made in accordance with the present invention. Apparatus 1 comprises inner and outer coaxial tubes. The inner tube is called the shaft 10 and the outer tube is called the sheath 40. Shaft 10 has proximal and distal ends 12 and 14 respectively. The distal end 14 of the shaft terminates at a luer lock hub 5. Preferably, shaft 10 has a proximal portion 16 which is made from a relatively stiff material such as stainless steel, Nitinol, or any other suitable material, and an distal portion 18 which is made from a polyethylene, polyimide, pellethane, Pebax, Vestamid, Cristamid, Grillamid or any other suitable material known to those of ordinary skill in the art. The two portions are joined together by any number of means known to those of ordinary skill in the art. The stainless steel proximal end gives the shaft the necessary rigidity or stiffness it needs to effectively push out the stent, while the polymeric distal portion provides the necessary flexibility to navigate tortuous vessels.

The distal portion 18 of the shaft has a distal tip 20 attached thereto. The distal tip 20 has a proximal end 34 whose diameter is substantially the same as the outer diameter of the sheath 40. The distal tip tapers to a smaller diameter from its proximal end to its distal end, wherein the distal end 36 of the distal tip has a diameter smaller than the inner diameter of the sheath. Also attached to distal portion 18 of shaft 10 is a stop 22 which is proximal to the distal tip 20. Stop 22 may be made from any number of materials known in the art, including stainless steel, and is even more preferably made from a highly radiopaque material such as platinum, gold tantalum. The diameter of stop 22 is substantially the same as the inner diameter of sheath 40, and would actually make frictional contact with the inner surface of the sheath. Stop 22 helps to push the stent out of the sheath during deployment, and helps the stent from migrating proximally into the sheath 40.

A stent bed 24 is defined as being that portion of the shaft between the distal tip 20 and the stop 22. The stent bed 24 and the stent 50 are coaxial so that the portion of shaft 18 comprising the stent bed 24 is located within the lumen of the stent 50. However, the stent bed 24 does not make any contact with stent 50 itself. Lastly, shaft 10 has a guidewire lumen 28 extending along its length from its proximal end 12 and exiting through its distal tip 20. This allows the shaft 10 to receive a guidewire much in the same way that an ordinary balloon angioplastly catheter receives a guidewire. Such guidewires are well known in art and help guide catheters and other medical devices through the vasculature of the body.

Sheath 40 is preferably a polymeric catheter and has a proximal end 42 terminating at a hub 152. Sheath 40 also has a distal end 44 which terminates at the proximal end 34 of distal tip 20 of the shaft 18, when the stent is in its fully un-deployed position as shown in the Figures. The distal end 44 of sheath 40 includes a radiopaque marker band 46 disposed along its outer surface. As will be explained below, the stent is fully deployed when the marker band 46 is lined up with radiopaque stop 22, thus indicating to the physician that it is now safe to remove the apparatus 1 from the body. Sheath 40 preferably comprises an outer polymeric layer and an inner polymeric layer. Positioned between outer and inner layers a braided reinforcing layer. Braided reinforcing layer is preferably made from stainless steel. The use of braided reinforcing layers in other types of medical devices can be found in US- 3,585,707 issued to Stevens on Jun. 22, 1971, US-5,045,072 issued to Castillo et al. on Sep. 3, 1991, and US-5,254,107 issued to Soltesz on Oct. 19, 1993.

Figures 1 and 2 show the stent 50 as being in its fully un-deployed position. This is the position the stent is in when the apparatus 1 is inserted into the vasculature and its distal end is navigated to a target site. Stent 50 is disposed around stent bed 24 and at the distal end 44 of sheath 40. The distal tip 20 of the shaft 10 is distal to the distal end 44 of the sheath 40, and the proximal end 12 of the shaft 10 is proximal to the proximal end 42 of the sheath 40. The stent 50 is in a compressed state and makes frictional contact with the inner surface 48 of the sheath 40.

When being inserted into a patient, sheath 40 and shaft 10 are locked together at their proximal ends by a Touhy Borst valve 8. This prevents any sliding movement between the shaft and sheath which could result in a premature deployment or partial deployment of the stent. When the stent 50 reaches its target site and is ready for deployment, the Touhy Borst valve 8 is opened so that that the sheath 40 and shaft 10 are no longer locked together.

The method under which apparatus 1 deploys stent 50 should be readily apparent. The apparatus 1 is first inserted into a vessel so that the stent bed 24 is at a target diseased site. Once this has occurred the physician would open the Touhy Borst valve 8. The physician would then grasp the proximal end 12 of shaft 10 so as to hold it in place. Thereafter, the physician would grasp the proximal end 42 of sheath 40 and slide it proximal, relative to the shaft 40. Stop 22 prevents the stent 50 from sliding back with the sheath 40, so that as the sheath 40 is moved back, the stent 50 is pushed out of the distal end 44 of the sheath 40. Stent deployment is complete when the radiopaque band 46 on the sheath 40 is proximal to radiopaque stop 22. The apparatus I may now be withdrawn through stent 50 and removed from the patient.

As described above, tapered stents better distribute stresses along the strut as opposed to the old design which has a high axial columnar stiffness, but which has its stresses concentrated proximate the loops. Each strut of a stent is designed to optimize the mechanical performance requirements of the stent while overcoming the size limitations imposed by the small profile of the blood vessels into which the stents are implanted.

The tapered strut design disclosed herein is directed toward maximizing the stent's axial and radial fatigue resistance, thus maximizing the stent's ability to resist the cyclical radial loading due to the pulsatile forces in the cardiovascular system as well as the axial, bending, crush and torsional loads due to forces external to the blood vessel in which the stent is implanted. These properties are crucial for stent performance since the overall fatigue resistance is vital for the long term integrity of the stent. The long term integrity of the stent, in turn, is crucial for providing the long term patentcy of the treated blood vessel.

While the tapered design for the struts spreads the strain distribution over the length of the struts, it also creates a potential for difficulties in loading the stent into the stent delivery system. Due to the tapering of the struts, larger gaps between struts remain when the stent is fully compressed/crimped as compared to the older straight strut design. Accordingly, when the compressed/crimped stent is pushed or loaded into the delivery system, the thinned struts may tend to buckle and/or twist which in turn may compress and/or deform the stent. This compression and/or deformation may further increase the loading resistance and may even potentially prevent the stent from being loaded onto the delivery system. In addition, increased resistance may be encountered during delivery of the stent into the vasculature if excessive deformation of the stent occurs.

While mating delivery system elements may be added in an alternative approach, it is extremely difficult to align the elements of the modified delivery system with the strut gaps.

In accordance with another exemplary embodiment, features may be added to the stent in localized areas, thereby increasing the pushability and/or column strength as well as the fatigue resistance of the stent while maintaining its flexibility during delivery and implantation, and without loosing any of the benefit created by the tapered strut design as described herein.

Referring to Figures 6A and 6B, there is illustrated a first exemplary embodiment of a stent segment 600 having an improved tapered strut design, shown in both the "as cut" state (Figure 6A) and the "as crimped" state (Figure 6B), in accordance with the present invention. The differences in shape between the stent segments illustrated in Figures 6A and 6B are due to the change in diameter and associated forces acting thereon. The shape of the strut in one state, for example, "as cut" is sufficient to fully describe the shape and all other design parameters of the stent. In this first exemplary embodiment, one set of tapered struts 602 comprises no protrusions, while a second set of tapered struts 604 comprises protrusions 606. Essentially, in this first exemplary embodiment, every other strut comprises a protrusion 606 located substantially midway between the strut ends both on the superior and inferior surfaces of the strut itself.

The protrusions 606 may comprise any suitable shape and or configuration. In the illustrated first exemplary embodiment, the protrusions 606 extend from both sides of the struts 604 like wings. The length of the extension of each protrusion 606 is approximately equal to the distance created by the gap 608 in the compressed state between adjacent tapered struts 602, 604 where the gap is the greatest. In other words, it is the gap created where each tapered strut is at its thinnest. In this way, the protrusions 606 will prevent side-to-side movement and thus will produce uniform and pushable stent elements. While this design prevents side-to-side strut movement, it also improves the fatigue resistance of the stent as evidenced by finite element analysis as described in detail below.

The protrusions 606 are preferably positioned at or near the center or thinnest section of the tapered struts 604. If the taper is not uniform, the protrusions 606 may be positioned at the thinnest point and not necessarily at the center. The protrusions 606 may be elements added after the stent is cut or most preferably, be cut out of the tube when all other elements and/or features of the stent are cut.

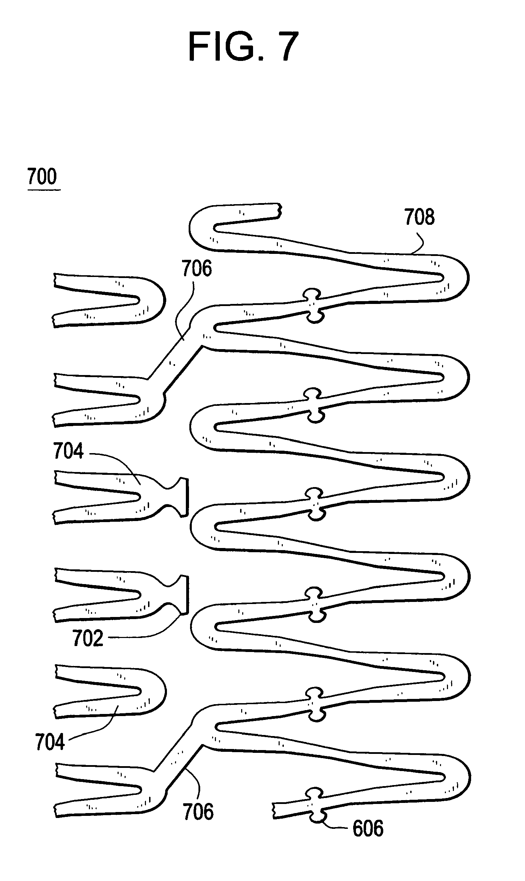

In accordance with another exemplary embodiment, extended tips or protrusions may be added to one or more of the loops not having bridges connecting adjacent hoops. Referring to Figure 7, there is illustrated a stent segment 700 having the protrusions 606 described above with respect to Figures 6A and 6B and extended tips 702. The extended tips 702 function to minimize axial compression upon loading, if required. As illustrated, only the loops 704 not having bridges 706 have the extended tips 702. Although, the extended tips 702 are only on every other hoop 708 so that each extended tip 702 makes contact with an adjacent loop 704 and not any other extended tip 702 it is envisioned that the extended tip 702 may be on either hoop and may even be present on both hoops if desired. The extended tips 702 may comprise any suitable configuration. In the exemplary embodiment, the tips 702 comprises a substantially anvil shape. In addition, as with the protrusions 606, the extended tips 702 may be a feature that is added to the loop or most preferably, laser cut from the same tube as every other element and/or feature.

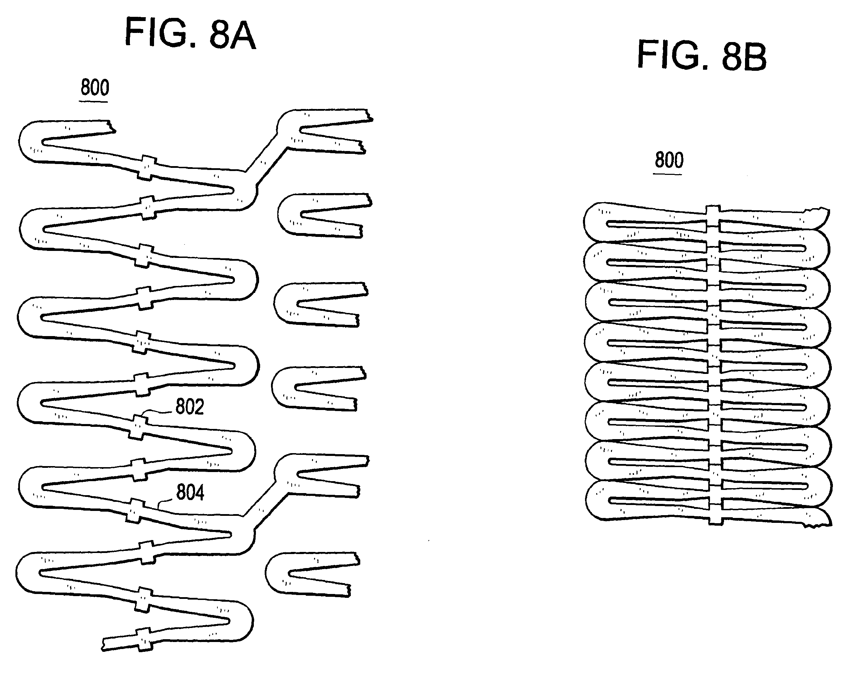

In accordance with yet another exemplary embodiment, protrusions may be added to all of the tapered struts, in contrast with the device illustrated in Figures 6A and 6B. Figures 8A and 8B illustrate this exemplary embodiment of the stent segment 800 in both the "as cut" state (Figure 8A) and the "as crimped" state. In this exemplary embodiment, the protrusions 802 are smaller in circumferential size as compared to those in Figures 6A and 6B. The protrusions 802 are smaller since they are on each of the tapered struts 804 and arranged so that they make contact with one another as illustrated in Figure 8B. Given that they make contact with one another, they can be approximately half the size of those illustrated in Figures 6A and 6B and cover the equivalent distance of the gap created between tapered struts, yet still provide the same functionality.

It is interesting to note that in performing finite element analysis of the stents illustrated in Figure 6A, 6B and Figures 8A, 8B, the maximum alternating strain which is a measure of fatigue resistance for a -5 percent to a +5 percent axial fatigue loading is 0.26 percent as compared to 0.27 percent for a tapered strut with no protrusions under equivalent loading. The lower the alternating strain of the device, the more fatigue resistant it becomes. From this analysis, it appears that while the tapered strut design described herein maximizes the stent's axial and radial fatigue resistance as compared to a straight strut, there may also be additional increase in fatigue resistance by adding the protusions which also relieves the problem of loading by increasing the column strength of the stent construct as described in detail herein.

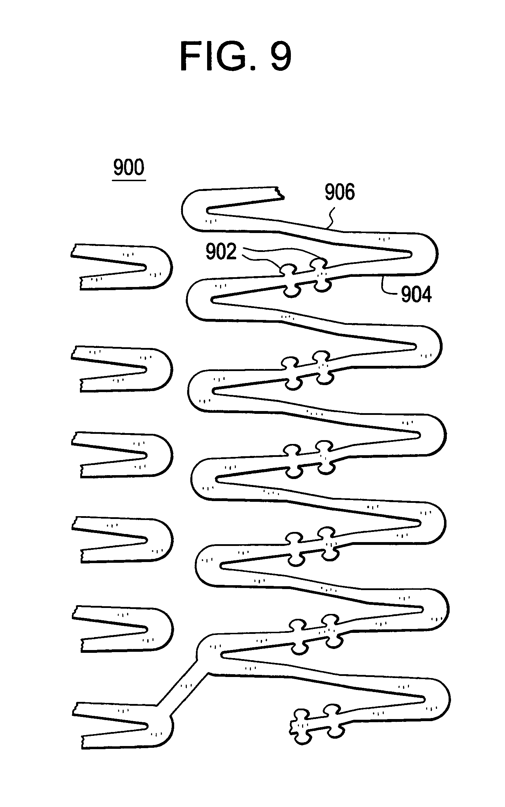

In accordance with yet another alternate exemplary embodiment, multiple protrusions may be positioned one or more struts. Figure 9 illustrates a stent segment 900 having two wing shaped protrusions 902 extending from every other strut 904. The remaining struts 906 have no protrusions. Essentially, this exemplary embodiment is identical to the design illustrated in Figures 6A and 6B except for the number of protrusions and their spacing. Given that there are two protrusions 902, they are spaced apart and not centered as in the device illustrated in Figures 6A and 6B. More than one protrusion 902 per stent 904 may reduce the maximum alternating strain making the device more fatigue resistant.

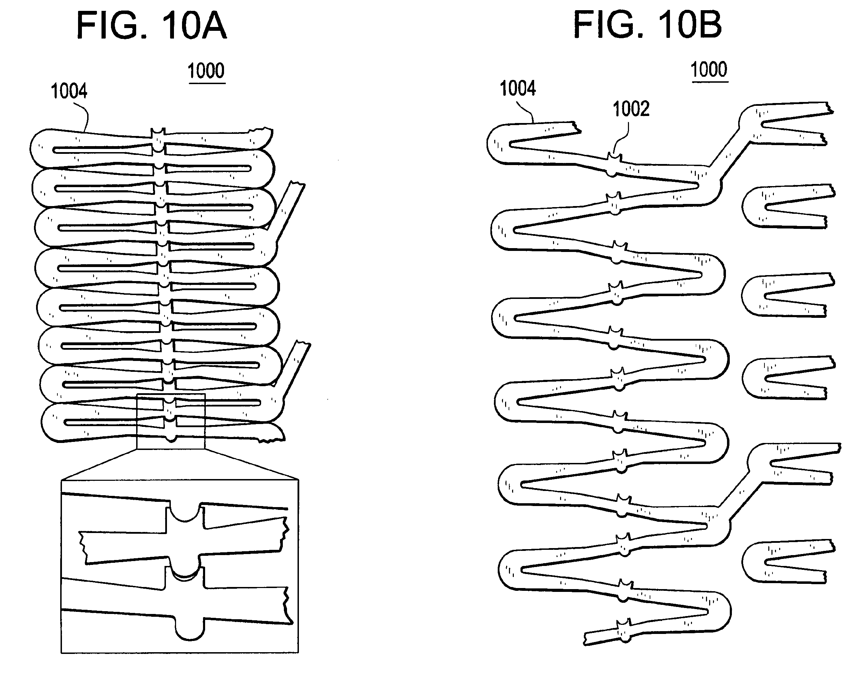

In all of the above described devices, the addition of protrusion(s) addresses the difficulties associated with the tapered strut design as well as slightly increasing the fatigue resistance which may be further increased utilizing multiple protrusions. In alternate exemplary embodiments, the protrusions may take on other shapes and/or configurations. For example, the protrusions may interlock or lock onto the adjacent tapered struts. Referring to Figures 10A and 10B, there is illustrated this exemplary embodiment. As illustrated, a stent segment 1000 has interlocking protrusions 1002. Figure 10 illustrates the stent 1004 in the compressed state and Figure 10B illustrates the stent 1004 in the expanded state.

## Claims

1. An intraluminal device for maintaining vessel patency comprising a substantially tubular structure having a first diameter for insertion into a vessel and a second diameter for deployment in a vessel, the substantially tubular structure being formed from a plurality of hoops, wherein adjacent hoops are connected by one or more bridges, each hoop comprising a plurality of longitudinally arranged struts (604) each strut having two opposing ends and a center therebeween, the width of the struts being greater at its opposing ends than at its center, and a plurality of loops connecting the plurality of struts to form a substantially s-shaped pattern, and **characterised in that** one or more struts in each hoop comprises an one or more circumferentially extending protrusions (606) proximate the center thereof.

2. The intraluminal device according to claim 1, wherein the struts (604) continuously taper from a greater width at its ends to a smaller width at its center.

3. The intraluminal device according to claim 1, wherein the one or more circumferentially extending protrusions are configured to abut one another on adjacent struts when the intraluminal device is in a crimped state.

4. The interaluminal device according to claim 1, wherein the one or more circumferentially extending protrusions are configured to be proximate one another on adjacent stents when the intraluminal device is in a crimpled state.

5. The intraluminal device according to claim 1, wherein the one or more circumferentially extending protrusions are configured to abut adjacent struts when the intraluminal device is in a crimped state.

6. The intraluminal device according to claim 1 further comprising one or more extending tips, the one or more extending tips are attached to one or more of the plurality of loops.

7. The intraluminal device according to claim 6, wherein the one or more extending tips comprise a substantially anvil type shape.

8. The intraluminal device according to claim 1, wherein the substantially tubular structure is a stent.

9. The intraluminal device according to claim 8, wherein the stent comprises a nickel-titanium alloy that exhibits superelastic properties at body temperature.

10. The intraluminal device according to claim 3, wherein the one or more circumferentially extending protrusions interlock with one another on adjacent struts when the intraluminal device is in a crimped state.

11. The intraluminal device according to claim 4, wherein the one or more circumferentially extending protrusions interlock with one another on adjacent struts when the intraluminal device is in a crimped state.

**Patentansprüche**

1. Intraluminale Vorrichtung zum Aufrechterhalten einer Gefäßdurchgängigkeit, die eine im Wesentliche röhrenförmige Struktur mit einem ersten Durchmesser zum Einführen in ein Gefäß und einem zweiten Durchmesser zum Entfalten in einem Gefäß umfasst, wobei die im Wesentlichen röhrenförmige Struktur aus mehreren Bändern gebildet ist, wobei benachbarte Bänder durch eine oder mehrere Brükken verbunden sind, jedes Band mehrere longitudinal angeordnete Streben (604) umfasst, jede Strebe zwei gegenüberliegende Ende und eine Mitte zwischen diesen umfasst, die Breite der Streben an ihren entgegen gesetzten Enden größer ist als in ihrer Mitte, und mehrere Schleifen die mehreren Streben verbinden, so dass ein im Wesentlichen S-förmiges Muster gebildet wird, und **dadurch gekennzeichnet, dass** eine oder mehrere Streben in jedem Band einen oder mehrere sich in Umfangsrichtung erstrekkende Vorsprünge (606) nahe der Mitte von diesen umfassen.

2. Intraluminale Vorrichtung nach Anspruch 1, wobei die Streben (604) sich kontinuierlich von einer größeren Breite an ihren Enden zu einer schmaleren Breite in ihrer Mitte verjüngen.

3. Intraluminale Vorrichtung nach Anspruch 1, wobei der eine oder die mehreren sich in Umfangsrichtung erstreckenden Vorsprünge dafür eingerichtet sind, an einem anderen auf einer benachbarten Strebe anzustoßen, wenn die intraluminale Vorrichtung sich in einem eingefalteten Zustand befindet.

4. Intraluminale Vorrichtung nach Anspruch 1, wobei der eine oder die mehreren sich in Umfangsrichtung erstreckenden Vorsprünge dafür eingerichtet sind, neben einer anderen auf einer benachbarten Strebe zu liegen, wenn die intraluminale Vorrichtung sich in einem eingefalteten Zustand befindet.

5. Intraluminale Vorrichtung nach Anspruch 1, wobei der eine oder die mehreren sich in Umfangsrichtung erstreckenden Vorsprünge dafür eingerichtet sind, an benachbarten Streben anzustoßen, wenn sich die intraluminale Vorrichtung in einem zusammengefalteten Zustand befindet.

6. Intraluminale Vorrichtung nach Anspruch 1, die weiterhin eine oder mehrere sich erstreckende Spitzen aufweist, wobei die eine oder die mehreren sich erstreckenden Spitzen an einer oder mehreren der mehreren Schleifen angebracht sind.

7. Intraluminale Vorrichtung nach Anspruch 6, wobei die eine oder die mehreren sich erstreckenden Spitzen eine im Wesentlichen ambosartige Form aufweisen.

8. Intraluminale Vorrichtung nach Anspruch 1, wobei die im Wesentliche röhrenförmige Struktur ein Stent ist.

9. Intraluminale Vorrichtung nach Anspruch 8, wobei der Stent eine Nickel-TitanLegierung umfasst, welche superelastische Eigenschaften bei Körpertemperatur aufweist.

10. Intraluminale Vorrichtung nach Anspruch 3, wobei der eine oder die mehreren sich in Umfangsrichtung erstreckenden Vorsprünge mit einem anderen auf einer benachbarten Streben ineinandergreifen, wenn die intraluminale Vorrichtung sich in einem zusammengefalteten Zustand befindet.

11. Intraluminale Vorrichtung nach Anspruch 4, wobei der eine oder die mehreren sich in Umfangsrichtung erstreckenden Vorsprünge mit einem anderen auf einer benachbarten Strebe ineinandergreifen, wenn sich die intraluminale Vorrichtung in einem gekrümmten Zustand befindet.

**Revendications**

1. Dispositif intraluminal pour maintenir la perméabilité d'un vaisseau comprenant une structure sensiblement tubulaire ayant un premier diamètre pour l'insertion dans un vaisseau et un deuxième diamètre pour le déploiement dans un vaisseau, la structure sensiblement tubulaire étant formée à partir d'une pluralité de cercles, dans lequel les cercles adjacents sont raccordés par un ou plusieurs ponts, chaque cercle comprenant une pluralité d'entretoises (604) agencées de manière longitudinale, chaque entretoise ayant deux extrémités opposées et un centre entre elles, la largeur des entretoises étant plus importante au niveau de leurs extrémités opposées qu'au niveau de leur centre, et une pluralité de boucles raccordant la pluralité d'entretoises afin de former un modèle sensiblement en forme de S, et **caractérisé en ce que** les unes ou plusieurs entretoises dans chaque cercle comprennent une ou plusieurs saillies (606) s'étendant de manière circonférentielle à proximité de leur centre.

2. Dispositif intraluminal selon la revendication 1, dans lequel les entretoises (604) se rétrécissent progressivement de manière continue à partir d'une largeur plus importante au niveau de leurs extrémités jusqu'à une largeur moins importante au niveau de leur centre.

3. Dispositif intraluminal selon la revendication 1, dans lequel les une ou plusieurs saillies s'étendant de manière circonférentielle sont configurées pour venir en butée les unes contre les autres sur les entretoises adjacentes lorsque le dispositif intraluminal est dans un état serti.

4. Dispositif intraluminal selon la revendication 1, dans lequel les une ou plusieurs saillies s'étendant de manière circonférentielle sont configurées pour être à proximité les unes des autres sur des stents adjacents lorsque le dispositif intraluminal est dans un état serti.

5. Dispositif intraluminal selon la revendication 1, dans lequel les une ou plusieurs saillies s'étendant de manière circonférentielle sont configurées pour venir en butée contre des entretoises adjacentes lorsque le dispositif intraluminal est dans un état serti.

6. Dispositif intraluminal selon la revendication 1, comprenant en outre une ou plusieurs pointes d'extension, les une ou plusieurs pointes d'extension sont fixées à une ou plusieurs boucles de la pluralité de boucles.

7. Dispositif intraluminal selon la revendication 6, dans lequel les une ou plusieurs pointes d'extension sont d'un type sensiblement en forme d'enclume.

8. Dispositif intraluminal selon la revendication 1, dans lequel la structure sensiblement tubulaire est un stent.

9. Dispositif intraluminal selon la revendication 8, dans lequel le stent comprend un alliage de nickel-titane qui laisse apparaître des propriétés super élastiques à la température corporelle.

10. Dispositif intraluminal selon la revendication 3, dans lequel les une ou plusieurs saillies s'étendant de manière circonférentielle se bloquent entre elles sur les entretoises adjacentes lorsque le dispositif intraluminal est dans un état serti.

11. Dispositif intraluminal selon la revendication 4, dans lequel les une ou plusieurs saillies s'étendant de manière circonférentielle se bloquent entre elles sur les entretoises adjacentes lorsque le dispositif intraluminal est dans un état serti.

# FIG. 1

# FIG. 2

FIG. 3

# FIG. 4

# FIG. 4A

FIG. 5

FIG. 6B

600

608

FIG. 6A

600

602
602
602
602
602
602
602

604 606
604 606
604 606
604 606
604 606
606

# FIG. 7

700

708

706

704

704

702

704

706

606

FIG. 8B

FIG. 8A

# FIG. 9

# FIG. 10A

# FIG. 10B

EP 2 158 880 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4733665 A, Palmaz
- US 4665771 A
- US 4665905 A, Jervis
- US 4925445 A, Sakamoto
- US 3585707 A, Stevens
- US 5045072 A, Castillo
- US 5254107 A, Soltesz